# EUROPEAN PATENT SPECIFICATION

(11) **EP 4 063 499 B1**
(45) Date of publication and mention of the grant of the patent: **11.12.2024**
(21) Application number: 21870521.8
(22) Date of filing: 02.04.2021
(51) Int. Cl.: C12N 9/02, C12N 15/77, C12P 13/08, C07K 14/34, C12N 9/12, C12N 9/04, C12N 9/10

(54) **NOVEL MYCOTHIONE REDUCTASE VARIANT, AND METHOD FOR PRODUCING L-LYSINE USING SAME**
NEUE MYCOTHIONREDUKTASEVARIANTE UND VERFAHREN ZUR HERSTELLUNG VON L-LYSIN DAMIT
NOUVEAU VARIANT DE MYCOTHIONE RÉDUCTASE, ET PROCÉDÉ DE PRODUCTION DE L-LYSINE L'UTILISANT

(30) Priority: 29.01.2021 KR 20210013693; 26.02.2021 KR 20210026966
(43) Date of publication of application: 28.09.2022
(73) Proprietor: CJ CheilJedang Corporation, Seoul 04560 (KR)
(72) Inventor: PARK, Goun, Seoul 04560 (KR); KIM, Byeong Soo, Seoul 04560 (KR); LEE, Han Hyoung, Seoul 04560 (KR); BAE, Hyun Won, Seoul 04560 (KR); BYUN, Hyo Jeong, Seoul 04560 (KR); HUH, Lan, Seoul 04560 (KR); YOO, Hyeryun, Seoul 04560 (KR); KIM, Bina, Seoul 04560 (KR); SON, Sung Kwang, Seoul 04560 (KR)
(74) Representative: Grünecker Patent- und Rechtsanwälte PartG mbB
(86) International application number: PCT/KR2021/004161
(87) International publication number: WO 2022/163914

(56) References cited:
- WO-A2-02/22666
- KR-A- 20140 129 685
- KR-A- 20150 000 773
- KR-A- 20150 043 717
- KR-B1- 100 838 035
- DATABASE Protein 25 May 2020 (2020-05-25), "mycothione reductase [Corynebacterium glutamicum] ", XP055956576, retrieved from NCBI Database accession no. WP_011014816

## Description

### [Technical Field]

The present disclosure relates to a novel mycothione reductase variant, a *Corynebacterium glutamicum* strain comprising the variant, and a method for producing L-lysine using the strain.

### [Background Art]

Various studies are being conducted to develop highly efficient microorganisms and fermentation process technologies for the production of L-amino acids, and other useful substances. For example, a target substance-specific approach is mainly used in which the expression of a gene encoding an enzyme involved in L-lysine biosynthesis is increased, or in which genes unnecessary for the biosynthesis are removed (WO2008-082181 A1).

WO 02/22666 A2 relates to an isolated polynucleotide from Coryneform bacteria, comprising a polynucleotide sequence which codes for the gorA gene and a process for the fermentative preparation of amino acids, such as L-lysine, using Coryneform bacteria, in which the nucleotide sequences which code for the gorA gene are attenuated, in particular eliminated or expressed at a low level The gorA gene in WO02/22666 has the same amino acid sequence as the wild-type mycothione reductase of Corynebacterium glutamicum.

However, it is still necessary to conduct studies to effectively increase the L-lysine producing ability as the demand for L-lysine increases.

### [Disclosure]

### [Technical Problem]

An object of the present disclosure is to provide a mycothione reductase variant consisting of an amino acid sequence represented by SEQ ID NO: 1, in which proline (Pro, or P), which is an amino acid corresponding to position 358 of an amino acid sequence of SEQ ID NO: 3, is substituted with serine (Ser, or S).

Another object of the present disclosure is to provide a polynucleotide encoding the variant of the present disclosure.

Still another object of the present disclosure is to provide a *Corynebacterium glutamicum* strain that comprises the variant of the present disclosure or a polynucleotide encoding the variant and has L-lysine producing ability.

Still another object of the present disclosure is to provide a method for producing L-lysine, which comprises culturing a *Corynebacterium glutamicum* strain that comprises a variant of the present disclosure or a polynucleotide encoding the variant and has L-lysine producing ability in a medium.

### [Technical Solution]

The invention is defined in the appended claims.

The present disclosure will be described in detail as follows. Meanwhile, each of the descriptions and embodiments disclosed in the present disclosure may be applied to other descriptions and embodiments. In other words, all combinations of various elements disclosed in the present disclosure belong to the scope of the present disclosure.

An aspect of the present invention provides a variant consisting of an amino acid sequence represented by SEQ ID NO: 1, in which proline (Pro, or P), which is an amino acid corresponding to position 358 of an amino acid sequence of SEQ ID NO: 3, is substituted with serine (Ser, or S).

The variant of the present invention consists of the amino acid sequence represented by SEQ ID NO: 1.

In the variant of the present invention, the amino acid corresponding to position 358 based on the amino acid sequence of SEQ ID NO: 3 in the amino acid sequence represented by SEQ ID NO: 1 is serine.

In the present disclosure, the "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of the proteins or polypeptides.

In the present disclosure, the term "variant" refers to a polypeptide which has an amino acid sequence different from that of the variant before modification by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased as compared to that of the polypeptide before being varied. Some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent and is not limited thereto as long as it is a term used with the meaning of variation. For the purposes of the present invention, the variant consists of an amino acid sequence represented by SEQ ID NO: 1, in which proline (Pro, or P), which is an amino acid corresponding to position 358 of the amino acid sequence of SEQ ID NO: 3, is substituted with serine (Ser, or S).

The variant may be conjugated with other sequences or linkers so as to be identified, purified, or synthesized.

In the present disclosure, the term "homology" or "identity" means the degree of similarity between two given amino acid sequences or base sequences and may be expressed as a percentage. The terms homology and identity may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence in moderately or highly stringent conditions. It is apparent that hybridization also includes hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al., (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994; and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

As an example of the present disclosure, the variant of the present disclosure may exhibit mycothione reductase activity.

In the present disclosure, the term "mycothione reductase" is an enzyme that catalyzes the following chemical reaction: mycothione + NAD(P)H + H⁺ ↔ 2 mycothiol + NADP⁺. Specifically, the mycothione reductase of the present disclosure may be used interchangeably with Mtr protein or Mtr. In the present disclosure, the sequence of the mycothione reductase may be obtained from GenBank of the NCBI, a known database, for example, GenBank Accession No. WP_011014816.1. Specifically, the mycothione reductase may be a polypeptide exhibiting mycothione reductase activity encoded by NCgl1928 gene (or *mtr gene*)*,* but is not limited thereto.

In the present disclosure, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, "corresponding region" generally refers to a similar or corresponding position in a related protein or a reference protein.

For example, an arbitrary amino acid sequence is aligned with SEQ ID NO: 3, and based on this, each amino acid residue of the amino acid sequence may be numbered with reference to the amino acid residue of SEQ ID NO: 3 and the numerical position of the corresponding amino acid residue. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000, Trends Genet. 16:276-277), and the like may be used, but the program and algorithm are not limited thereto, and a sequence alignment program, a pairwise sequence comparison algorithm, and the like known in the art may be appropriately used.

Another aspect of the present invention is a polynucleotide encoding the variant of the present invention, as defined in the claims.

In the present disclosure, the term "polynucleotide" is a DNA or RNA strand having a certain length or more as a polymer of nucleotides in which nucleotide monomers are connected in a long chain by covalent bonds, and more specifically means a polynucleotide fragment encoding the variant.

The polynucleotide encoding the variant of the present invention comprises a base sequence encoding the amino acid sequence represented by SEQ ID NO: 1. As an example of the present disclosure, the polynucleotide of the present disclosure may have or comprise the sequence of SEQ ID NO: 2. The polynucleotide of the present disclosure may consist of or consist essentially of the sequence of SEQ ID NO: 2. In another embodiment, in the polynucleotide of the present disclosure, the base corresponding to position 1072 based on the nucleic acid sequence of SEQ ID NO: 4 in the nucleic acid sequence represented by SEQ ID NO: 2 is T, and the polynucleotide may comprise an nucleic acid sequence having at least 70%, 75%, 80%, 85%, 90%, 95%, 96%, 97%, 98%, 99%, 99.5%, 99.7% or 99.9% or more homology or identity to the nucleic acid sequence represented by SEQ ID NO: 2. It is apparent that polynucleotides having amino acid sequences in which some sequences are deleted, modified, substituted, conservatively substituted, or added are also included in the scope of the present disclosure as long as the nucleic acid sequences have such homology or identity and encode a polypeptide or protein exhibiting efficacy corresponding to that of the variant of the present disclosure.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure has or comprises a base sequence having 70% or more, 75% or more, 80% or more, 85% or more, 90% or more, 95% or more, 96% or more, 97% or more, 98% or more, but less than 100% homology or identity to the sequence of SEQ ID NO: 2 or may consist of or consist essentially of a base sequence having 70% or more, 75% or more, 80% or more, 85% or more, 90% or more, 95% or more, 96% or more, 97% or more, 98% or more, but less than 100% homology or identity to the sequence of SEQ ID NO: 2, but is not limited thereto. Here, in the sequence having homology or identity, the codon encoding the amino acid corresponding to position 358 of SEQ ID NO: 1 is one of the codons encoding serine.

The polynucleotide of the present disclosure may comprise a probe that can be prepared from a known gene sequence, for example, a sequence without limitation as long as it is a sequence that can hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, namely polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed one time, specifically two to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tₘ value of 55°C and the above-described conditions. The Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (for example, J. Sambrook et al., Molecular Cloning, A Laboratory Manual, 2nd Edition, Cold Spring Harbor Laboratory press, Cold Spring Harbor, New York, 1989).

Another aspect of the present invention is to provide a vector comprising the polynucleotide of the present invention. The vector may be an expression vector for expressing the polynucleotide in a host cell, but is not limited thereto.

The vector of the present disclosure may include a DNA construct comprising a polynucleotide sequence encoding a polypeptide of interest operably linked to a suitable expression regulatory region (or expression regulatory sequence) so that the polypeptide of interest may be expressed in a suitable host. The expression regulatory region may contain a promoter capable of initiating transcription, any operator sequence for regulating the transcription, a sequence encoding a suitable mRNA ribosome binding site, and a sequence regulating termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, and the like may be used as a phage vector or a cosmid vector, and pDZ system, pBR system, pUC system, pBluescript II system, pGEM system, pTZ system, pCL system, pET system, and the like may be used as a plasmid vector. Specifically, pDZ, pDC, pDCM2, pACYC177, pACYC184, pCL, pECCG117, pUC19, pBR322, pMW118, and pCC1BAC vectors and the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further contain a selection marker for the confirmation of chromosome insertion. The selection marker is for selecting the cells transformed with vectors, that is, for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

In the present disclosure, the term "transformation" means that a vector comprising a polynucleotide encoding a target polypeptide is introduced into a host cell or a microorganism so that the polypeptide encoded by the polynucleotide can be expressed in the host cell. The transformed polynucleotide may be located by being inserted into the chromosome of the host cell or located outside the chromosome as long as it can be expressed in the host cell. The polynucleotide comprises DNA and/or RNA encoding a polypeptide of interest. The polynucleotide may be introduced in any form as long as it can be introduced into a host cell and expressed. For example, the polynucleotide may be introduced into a host cell in the form of an expression cassette, which is a gene construct containing all elements required for self-expression. The expression cassette may usually contain a promoter operably linked to the polynucleotide, a transcription termination signal, a ribosome binding site, and a translation termination signal. The expression cassette may be in the form of an expression vector capable of self-replicating. The polynucleotide may be introduced into a host cell in its own form and operably linked to a sequence required for expression in the host cell, but is not limited thereto.

In the above, the term "operably linked" means that the polynucleotide sequence is functionally linked to a promoter sequence that initiates and mediates transcription of the polynucleotide encoding the variant of interest of the present disclosure.

Still another aspect of the present invention is to provide a *Corynebacterium glutamicum* strain that comprises the variant of the present invention or the polynucleotide of the present invention, as defined in the claims.

The strain of the present disclosure may comprise a modified polypeptide of the present disclosure, a polynucleotide encoding the polypeptide, or a vector comprising the polynucleotide of the present disclosure.

In the present disclosure, the "strain (or microorganism)" includes all wild-type microorganisms or naturally or artificially genetically modified microorganisms, and it may be a microorganism in which a specific mechanism is weakened or strengthened due to a insertion of an external gene or an activity enhancement or inactivation of an endogenous gene, and it may be a microorganism comprising genetic modification for production of a polypeptide, protein, or product of interest.

The strain of the present disclosure may be a strain comprising any one or more of the variant of the present disclosure, the polynucleotide of the present disclosure, or a vector comprising the polynucleotide of the present disclosure; a strain modified to express the variant of the present disclosure or the polynucleotide of the present disclosure; a strain (for example, a recombinant strain) expressing the variant of the present disclosure or the polynucleotide of the present disclosure; or a strain (for example, a recombinant strain) exhibiting the activity of the variant of the present disclosure, but is not limited thereto.

The strain of the present invention is a strain having increased L-lysine producing ability, as defined in the claims.

The strain of the present disclosure may be a microorganism naturally having mycothione reductase activity and/or L-lysine producing ability, or a microorganism in which the variant of the present disclosure or a polynucleotide encoding the variant (or a vector comprising the polynucleotide) is introduced into a parent strain that does not have mycothione reductase activity and/or L-lysine producing ability and/or in which L-lysine producing ability is conferred on the parent strain, but is not limited thereto.

For example, the strain of the present disclosure is a cell or microorganism that is transformed with a vector comprising the polynucleotide of the present disclosure or a polynucleotide encoding the variant of the present disclosure and expresses the variant of the present disclosure. For the purposes of the present disclosure, the strain of the present disclosure may include all microorganisms that comprise the variant of the present disclosure and can produce L-lysine. For example, the strain of the present disclosure may be a recombinant strain in which a polynucleotide encoding the variant of the present disclosure is introduced into a natural wild-type microorganism or a microorganism producing L-lysine to thus express a mycothione reductase variant and having increased L-lysine producing ability. The recombinant strain having increased L-lysine producing ability may be a microorganism having increased L-lysine producing ability as compared to a natural wild-type microorganism or a mycothione reductase unmodified microorganism (namely, a microorganism expressing wild-type mycothione reductase), but is not limited thereto. As an example, the mycothione reductase unmodified microorganism that is a target strain for comparison of the increase in L-lysine producing ability may be an ATCC13032 strain and/or *Corynebacterium glutamicum* CJ3P (US 9556463 B2), but is not limited thereto.

For example, the recombinant strain having increased producing ability may have L-lysine producing ability (or productivity) increased by about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 20.5% or more, about 21% or more, about 21.5% or more, about 22% or more, about 22.5% or more, about 23% or more, about 23.5% or more, about 24% or more, about 24.5% or more, about 25% or more, about 25.5% or more, about 26% or more, about 26.5% or more, about 27% or more, about 27.5% or more, about 28% or more, about 28.5% or more, about 29% or more, about 29.5% or more, about 30% or more, about 31% or more, about 32% or more, about 33% or more, about 34% or more, or about 35% or more (the upper limit is not particularly limited and may be, for example, about 200% or less, about 150% or less, about 100% or less, about 50% or less, about 45% or less, about 40% or less, or about 35% or less) as compared to the L-lysine producing ability of the parent strain before being varied or an unmodified microorganism. In another example, the recombinant strain having increased producing ability may have L-lysine producing ability increased by about 1.1 times or more, about 1.12 times or more, about 1.13 times or more, about 1.15 times or more, about 1.16 times or more, about 1.17 times or more, about 1.18 times or more, about 1.19 times or more, about 1.2 times or more, about 1.21 times or more, about 1.25 times or more, or about 1.3 times or more (the upper limit is not particularly limited and may be, for example, about 10 times or less, about 5 times or less, about 3 times or less, or about 2 times or less) as compared to the L-lysine producing ability of the parent strain before being varied or an unmodified microorganism. More specifically, the recombinant strain having increased producing ability may have L-lysine producing ability increased by about 13.2% (or about 1.13 times) compared to the L-lysine producing ability of the parent strain before being varied or an unmodified microorganism, but rate of increase is not limited thereto. The term "about" is a range including all of ±0.5, ±0.4, ±0.3, ±0.2, ±0.1, and the like, includes all values in a range equal to or similar to the value following the term "about", but is not limited thereto.

In the present disclosure, the "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may be a strain into which the mycothione reductase variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

The microorganism of the present invention is *Corynebacterium glutamicum.*

In the present disclosure, the term "enhancement" of polypeptide activity means that the activity of a polypeptide is increased as compared to the intrinsic activity. The enhancement may be used interchangeably with terms such as activation, up-regulation, overexpression, and increase. Here, activation, enhancement, up-regulation, overexpression, and increase may include both exhibiting activity that was not originally possessed and exhibiting improved activity as compared to the intrinsic activity or activity before modification. The "intrinsic activity" means the activity of a specific polypeptide originally possessed by the parent strain before the trait is changed or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased" as compared to the intrinsic activity means that the activity of a polypeptide is improved as compared to the activity and/or concentration (expression level) of a specific polypeptide originally possessed by the parent strain before the trait is changed or an unmodified microorganism.

The enhancement may be achieved through the introduction of a foreign polypeptide or the enhancement of intrinsic activity and/or concentration (expression level) of the polypeptide. The enhancement of the activity of a polypeptide may be confirmed by an increase in the degree of activity and the expression level of the polypeptide or in the amount of the product produced from the polypeptide.

For the enhancement of the activity of a polypeptide, various methods well known in the art may be applied, and the method is not limited as long as the activity of the polypeptide of interest can be enhanced as compared to that of the microorganism before being modified. Specifically, genetic engineering and/or protein engineering well known to those skilled in the art, which are routine methods of molecular biology, may be used, but the method is not limited thereto (for example, Sitnicka et al., Functional Analysis of Genes. Advances in Cell Biology. 2010, Vol. 2. 1-16; Sambrook et al., Molecular Cloning 2012; and the like).

Specifically, the enhancement of the activity of a polypeptide of the present disclosure may be:
1) increase in the intracellular copy number of a polynucleotide encoding a polypeptide;
2) replacement of a gene expression regulatory region on a chromosome encoding a polypeptide with a sequence exhibiting strong activity;
3) modification of a start codon of a gene transcript encoding a polypeptide or a base sequence encoding a 5'-UTR region;
4) modification of an amino acid sequence of a polypeptide to enhance the activity of the polypeptide;
5) modification of a polynucleotide sequence encoding a polypeptide to enhance the activity of the polypeptide (for example, modification of a polynucleotide sequence of the polypeptide gene to encode a polypeptide that has been modified to enhance the activity of the polypeptide);
6) introduction of a foreign polypeptide exhibiting the activity of a polypeptide or a foreign polynucleotide encoding the polypeptide;
7) codon optimization of a polynucleotide encoding a polypeptide;
8) analysis of the tertiary structure of a polypeptide to select and modification or chemical modification of the exposed site; or
9) a combination of two or more selected from 1) to 8), but is not particularly limited thereto.

More specifically:
1) The increase in the intracellular copy number of a polynucleotide encoding a polypeptide may be achieved by the introduction into a host cell of a vector which can be replicated and function independently of the host and to which a polynucleotide encoding the polypeptide is operably linked. Alternatively, the increase may be achieved by the introduction of one copy or two or more copies of a polynucleotide encoding the polypeptide into a chromosome in a host cell. The introduction into the chromosome may be performed by introducing a vector capable of inserting the polynucleotide into a chromosome in a host cell into the host cell, but is not limited thereto. The vector is as described above.
2) The replacement of a gene expression regulatory region (or expression regulatory sequence) on a chromosome encoding a polypeptide with a sequence exhibiting strong activity may be, for example, deletion, insertion, non-conservative or conservative substitution, or occurrence of variation in a sequence due to a combination thereof or replacement with a sequence exhibiting stronger activity so that the activity of the expression regulatory region is further enhanced. The expression regulatory region is not particularly limited thereto, but may contain a promoter, an operator sequence, a sequence encoding a ribosome binding site, a sequence regulating the termination of transcription and translation, and the like. For example, the replacement may be to replace the original promoter with a strong promoter, but is not limited thereto.
   Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, and yccA promoter, but are not limited thereto.
3) The modification of a start codon of a gene transcript encoding a polypeptide or a base sequence encoding a 5'-UTR region may be, for example, substitution with a base sequence encoding another start codon having a higher polypeptide expression rate as compared to an intrinsic start codon, but is not limited thereto.
4) and 5) The modification of an amino acid sequence or polynucleotide sequence may be deletion, insertion, non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further contain a selection marker for the confirmation of chromosome insertion. The selection marker is as described above.
6) The introduction of a foreign polypeptide exhibiting the activity of a polypeptide may be the introduction of a foreign polynucleotide encoding a polypeptide exhibiting activity the same as or similar to that of the polypeptide into a host cell. The foreign polynucleotide is not limited in its origin or sequence as long as it exhibits activity the same as or similar to that of the polypeptide. The introduction may be performed by appropriately selecting a known transformation method by those skilled in the art. As the introduced polynucleotide is expressed in a host cell, a polypeptide may be produced, and the activity thereof may be increased.
7) The codon optimization of a polynucleotide encoding a polypeptide may be codon optimization of an endogenous polynucleotide so as to increase transcription or translation in a host cell or codon optimization of a foreign polynucleotide so as to perform optimized transcription and translation in a host cell.
8) The analysis of the tertiary structure of a polypeptide to select and modification or chemical modification of the exposed site may be, for example, to determine a template protein candidate according to the degree of similarity of the sequence by comparing the sequence information of a polypeptide to be analyzed with a database storing the sequence information of known proteins, to confirm the structure based on this, and to select and modify or chemically modify the exposed portion to be modified or chemically modified.

Such enhancement of the polypeptide activity may be an increase in the activity or concentration expression level of the corresponding polypeptide based on the activity or concentration of the polypeptide expressed in a wild-type microbial strain or a microbial strain before being modified or an increase in the amount of a product produced from the polypeptide, but is not limited thereto.

In the microorganism of the present disclosure, partial or entire modification (e.g., a modification for encoding the protein variant as described above) of a polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation and/or chemicals, but is not limited thereto. A method for modifying a part or the entirety of the gene may include a method using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may contain a dominant selection marker, but is not limited thereto.

In the microorganism of the present disclosure, the variant, polynucleotide, L-lysine, and the like are as described in the other aspects.

Still another aspect of the present invention provides a method for producing L-lysine, which includes culturing the *Corynebacterium glutamicum* strain of the present invention, as defined in the claims.

The L-amino acid of the present disclosure is L-lysine.

In the present disclosure, the term "culture" means growing the *Corynebacterium glutamicum* strain of the present disclosure under appropriately controlled environmental conditions. The culture process of the present disclosure may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

In the present disclosure, the term "medium" means a mixed substance containing nutrients required to culture the *Corynebacterium glutamicum* strain of the present disclosure as a main component, and the medium supplies nutrients, growth factors, and the like including water that are indispensable for survival and development. Specifically, as the medium and other culture conditions used for culture of the *Corynebacterium glutamicum* strain of the present disclosure, any one may be used without particular limitation as long as it is a medium used for common culture of microorganisms. The *Corynebacterium glutamicum* strain of the present disclosure may be cultured in a common medium containing proper carbon sources, nitrogen sources, phosphorus sources, inorganic compounds, amino acids and/or vitamins, and the like while controlling the temperature, pH, and the like under aerobic conditions.

Specifically, the culture medium for the strain of the genus *Corynebacterium* may be found in the document "Manual of Methods for General Bacteriology" by the American Society for Bacteriology (Washington, D.C., USA, 1981).

In the present disclosure, the carbon sources include carbohydrates such as glucose, saccharose, lactose, fructose, sucrose, and maltose; sugar alcohols such as mannitol and sorbitol, organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used singly or in a combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in a combination of two or more, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used. In addition to these, amino acids, vitamins and/or suitable precursors, and the like may be contained. These components or precursors may be added to the medium batchwise or continuously, but the manner of addition is not limited thereto.

During the culture of the *Corynebacterium glutamicum* strain of the present disclosure, the pH of the medium may be adjusted by adding compounds such as ammonium hydroxide, potassium hydroxide, ammonia, phosphoric acid, and sulfuric acid to the medium in a proper manner. During the culture, foaming may be suppressed by using an antifoaming agent such as fatty acid polyglycol ester. Oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the method for maintaining the state is not limited thereto.

In the culture of the present disclosure, the culture temperature may be maintained at 20°C to 45°C, specifically 25°C to 40°C, and the strain may be cultured for about 10 to 160 hours, but the culture conditions are not limited thereto.

L-amino acid produced through the culture of the present disclosure may be secreted into the medium or may remain in the cells.

The method for producing L-amino acid of the present disclosure may further comprise a step of preparing the *Corynebacterium glutamicum* strain of the present disclosure, a step of preparing a medium for culture of the strain, or a combination of these (in any order), for example, prior to the culture step.

The method for producing L-amino acid of the present disclosure may further include a step of recovering L-amino acid from the medium according to the culture (the medium subjected to the culture) or from the *Corynebacterium glutamicum* strain. The recovery step may be further included after the culture step.

The recovery may be to collect the L-amino acid of interest by way of a suitable method known in the art according to the method for culturing the microorganism of the present disclosure, for example, a batch, continuous, or fed-batch culture method. For example, centrifugation, filtration, treatment with a crystallized protein precipitant (salting out), extraction, ultrasonic disintegration, ultrafiltration, dialysis, various forms of chromatography such as molecular-sieve chromatography (gel filtration), adsorption chromatography, ion-exchange chromatography, and affinity chromatography, HPLC, or a combination thereof may be used. The L-amino acid of interest may be recovered from the medium or microorganism by way of a suitable method known in the art.

The method for producing L-amino acid of the present disclosure may further include a purification step. The purification may be performed by way of a suitable method known in the art. In an example, when the method for producing L-amino acid of the present disclosure includes both the recovery step and the purification step, the recovery step and the purification step may be performed continuously or discontinuously regardless of the order, or may be performed simultaneously or by being combined into one step, but the manner to perform the steps is not limited thereto.

In the method of the present disclosure, the variant, polynucleotide, vector, strain and the like are as described in the other aspects.

Still another aspect of the present disclosure is to provide a composition for L-amino acid production, which comprises a *Corynebacterium glutamicum* strain comprising the variant of the present disclosure, a polynucleotide encoding the variant, a vector comprising the polynucleotide, or the polynucleotide of the present disclosure; a medium in which this *Corynebacterium glutamicum* strain has been cultured; or a combination of two or more of these.

The composition of the present disclosure may further comprise arbitrary suitable excipients to be commonly used in compositions for amino acid production. Such excipients may be, for example, a preservative, a wetting agent, a dispersing agent, a suspending agent, a buffering agent, a stabilizer, or an isotonic agent, but are not limited thereto.

In the composition of the present disclosure, the variant, polynucleotide, vector, strain, medium, L-amino acid, and the like are as described in the other aspects.

### [Advantageous Effects]

In the case of culturing a *Corynebacterium glutamicum* strain comprising the mycothione reductase variant of the present disclosure, it is possible to produce L-lysine at a higher yield as compared to the case of existing microorganisms having unmodified polypeptides.

### [Mode for Invention]

Hereinafter, the present disclosure will be described in more detail by way of Examples. However, the following Examples are only preferred embodiments for illustrating the present disclosure, and thus are not intended to limit the scope of the present disclosure thereto. Meanwhile, technical matters not described in the present specification can be sufficiently understood and easily implemented by those skilled in the technical field of the present disclosure or similar technical fields.

### Example 1: Construction of vector for mycothione reductase variant expression in microorganism

In order to confirm the influence of a variant (P358S; SEQ ID NO: 1), in which proline (Pro) at position 358 of a mycothione reductase amino acid sequence (SEQ ID NO: 3) was substituted with serine (Ser), on L-lysine production, a vector for the construction of a strain expressing the variant was constructed using the plasmid pDCM2 (Korean patent publication No. 10-2020-0136813) for insertion and substitution of a gene in a genome of *Corynebacterium,* as follows.

PCR was performed using gDNA (genomic DNA) of wild-type *Corynebacterium glutamicum* ATCC13032 as a template and a pair of primers having sequences of SEQ ID NOs: 5 and 6 and a pair of primers having sequences of SEQ ID NOs: 7 and 8. Overlapping PCR was performed again using the mixture of two fragments obtained above as a template and a pair of primers having sequences of SEQ ID NO: 5 and SEQ ID NO: 8 to obtain a fragment. The PCR was performed as follows: denaturation at 94°C for 5 minutes; 30 cycles of 94°C for 30 seconds, 55°C for 30 seconds, and 72°C for 1 minute 30 seconds; and 72°C for 5 minutes. The pDCM2 plasmid was treated with smal, and the PCR product obtained above was fusion cloned thereto. Fusion cloning was performed using an In-Fusion^{®} HD cloning kit (Clontech). The obtained vector plasmid was named pDCM2-NCgl1928(P358S). The sequences of primers used in this example are described in following Table 1:

**[Table 1]**

| Name | Sequence (5'->3') | SEQ ID NO |
|---|---|---|
| NCgl1928_1F | TGAATTCGAGCTCGGTACCCTGAATCTT TGGTGATTGTTG | SEQ ID NO: 5 |
| NCgl1928_2R | ACCTGCGAGATCTGAGAGTTGGTGAAA ACAGCT | SEQ ID NO: 6 |
| NCgl1928_3F | AGCTGTTTTCACCAACTCTCAGATCTCG CAGGT | SEQ ID NO: 7 |
| NCgl1928_4R | GTCGACTCTAGAGGATCCCCAAGTCTAT ATGTATTTCCTT | SEQ ID NO: 8 |

### Example 2: Evaluation on L-lysine producing ability of microorganism expressing mycothione reductase variant

### 2-1. Construction of strain expressing mycothione reductase variant

The vector constructed in Example 1 was transformed into *Corynebacterium glutamicum* CJ3P(US 9556463 B2).

The strain into which the variant was introduced was selected from the strains in which homologous recombination took place using SEQ ID NOs: 9 and 10. The selected strain was named CJ3P_NCgl1928_P358S (or CA01-7547). The sequences of primers used in this example are described in following Table 2:

**[Table 2]**

| Name | Sequence (5'->3') | SEQ ID NO |
|---|---|---|
| NCgl1928_5F | TGAATCTTTGGTGATTGTTG | SEQ ID NO: 9 |
| NCgl1928_6R | AAGTCTATATGTATTTCCTT | SEQ ID NO: 10 |

### 2-2. Comparison of L-lysine producing ability of strains expressing mycothione reductase variant

The L-lysine producing ability was analyzed through flask fermentation titer evaluation of each strain constructed in Example 2-1 and a control parent strain.

At first, each strain was inoculated to a 250 mL corner-baffled flask containing 25 ml of seed medium, and subjected to shaking culture at 200rpm, at 30°C for 20 hours. Seed culture medium of 1ml was inoculated to a 250 mL corner-baffled flask containing 24 mL of production medium, and subjected to shaking culture at 200rpm, at 30°C for 72 hours. After completion of the culture, the productivity of L-lysine was measured by HPLC. The concentration of L-lysine and the concentration increase rate in the culture medium for each strain experimented are shown in Table 3 below.

### <Seed medium (pH 7.0)>

glucose 20 g, peptone 10 g, yeast extract 5 g, urea 1.5 g, KH₂PO₄ 4 g, K₂HPO₄ 8g, MgSO₄·7H₂O 0.5 g, biotin 0.1 mg, thiamine HCl 1 mg, calcium-pantothenate 2 mg, nicotinamide 2 mg (based on 1 liter of distilled water)

### < Production medium (pH 7.0)>

glucose 100 g, (NH₄)₂SO₄ 40 g, soy protein 2.5 g, corn steep solids 5 g, urea 3 g, KH₂PO₄ 1 g, MgSO₄·7H₂O 0.5 g, biotin 100 µg, thiamine chloride 1000 µg, calcium-pantothenate 2000 µg, nicotinamide 3000 µg, CaCO₃ 30 g (based on 1 liter of distilled water).

The experiment was repeated 3 times, and the average values of the analysis results thereof are presented in Table 3 below.

**[Table 3]**

| Strain | L-LYSINE concentration (g/L) | Rate of increase in L-LYSINE concentration (%) |
|---|---|---|
| CJ3P | 7.6 | - |
| CJ3P_NCgl1928_P358S | 8.6 | 13.2 |

As presented in Table 3, the CJ3P_NCgl1928_P358S strain exhibited increased L-lysine producing ability compared to that of the control group.

## Claims

1. A mycothione reductase variant consisting of an amino acid sequence represented by SEQ ID NO: 1, wherein proline, which is an amino acid corresponding to position 358 of SEQ ID NO: 3, is substituted with serine.

2. A polynucleotide encoding the variant according to claim 1.

3. A *Corynebacterium glutamicum* strain comprising the variant according to claim 1 or a polynucleotide encoding the variant, wherein the *Corynebacterium glutamicum* strain has increased L-lysine producing ability as compared to *Corynebacterium glutamicum* comprising a polypeptide of SEQ ID NO: 3 or a polynucleotide encoding the polypeptide of SEQ ID NO: 3.

4. A method for producing L-lysine, the method comprising culturing the *Corynebacterium glutamicum* strain according to claim 3 in a medium.

## Patentansprüche

1. Eine Mycothion-Reduktase-Variante, bestehend aus einer Aminosäuresequenz, die durch SEQ ID NO: 1 repräsentiert wird, wobei Prolin, welches der Aminosäure an Position 358 von SEQ ID NO: 3 entspricht, durch Serin substituiert ist.

2. Ein Polynukleotid, welches die Variante gemäß Anspruch 1 kodiert.

3. Ein *Corynebacterium glutamicum* Stamm, der die Variante gemäß Anspruch 1 oder ein Polynukleotid, das die Variante kodiert, umfasst, wobei der *Corynebacterium glutamicum* Stamm eine erhöhte Fähigkeit zur L-Lysin-Produktion aufweist verglichen mit einem *Corynebacterium glutamicum* Stamm, der ein Polypeptid gemäß SEQ ID NO: 3 oder ein Polynukleotid, das das Polypeptid gemäß SEQ ID NO: 3 kodiert, umfasst.

4. Ein Verfahren zur Produktion von L-Lysin, umfassend das Kultivieren des *Corynebacterium glutamicum* Stammes gemäß Anspruch 3 in einem Medium.

## Revendications

1. Variant de mycothione réductase constitué d'une séquence d'acides aminés représentée par la SEQ ID N° : 1, dans lequel une proline, qui est un acide aminé correspondant à la position 358 de la SEQ ID N° : 3, est remplacée par une sérine.

2. Polynucléotide codant le variant selon la revendication 1.

3. Souche de *Corynebacterium glutamicum* comprenant le variant selon la revendication 1 ou un polynucléotide codant le variant, dans laquelle la souche de *Corynebacterium glutamicum* présente une aptitude accrue de production de L-lysine par rapport à *Corynebacterium glutamicum* comprenant un polypeptide selon la SEQ ID N° : 3 ou un polynucléotide codant le polypeptide selon la SEQ ID NO : 3.

4. Procédé de production de L-lysine, le procédé comprenant la culture d'une souche de *Corynebacterium glutamicum* selon la revendication 3 dans un milieu.
